# EUROPEAN PATENT SPECIFICATION

(11) **EP 4 736 197 B1**
(45) Date of publication and mention of the grant of the patent: **23.09.2026**
(21) Application number: 25722467.5
(22) Date of filing: 10.04.2025
(51) Int. Cl.: H01B 1/02, A61N 1/00

(54) **METHOD FOR THE PRODUCTION OF STRETCHABLE CONDUCTIVE DEVICES AND DEVICES THUS OBTAINED**
VERFAHREN ZUR HERSTELLUNG DEHNBARER LEITFÄHIGER VORRICHTUNGEN UND SO ERHALTENE VORRICHTUNGEN
PROCÉDÉ DE FABRICATION DE DISPOSITIFS CONDUCTEURS ÉTIRABLES ET DISPOSITIFS AINSI OBTENUS

(30) Priority: 11.04.2024 IT 202400008122
(43) Date of publication of application: 06.05.2026
(73) Proprietor: WISE S.P.A., 20093 Cologno Monzese (IT)
(72) Inventor: SAINI, Matteo, 20832 Desio (IT); FERRARI, Sandro, 24124 Bergamo (IT); SPREAFICO, Laura, 20030 Senago (IT); SADEGHPOUR, Arezoo, 26838 Tavazzano con Villavesco (IT)
(74) Representative: Palladino, Saverio Massimo
(86) International application number: PCT/EP2025/059840
(87) International publication number: WO 2025/215133

(56) References cited:
- US-B2- 7 085 605

## Description

### FIELD OF THE INVENTION

The present invention relates to a method for the production of devices consisting of an elastomeric polymeric support on which a metallic film is present, which maintains the electrical conductivity properties even in the elongation and relaxation cycles of the support. The invention further relates to stretchable conductive devices thus obtained.

### BACKGROUND ART

In many fields of the art, there is a need to establish a stable electrical connection by means of conductors which, in addition to being flexible, are stretchable, i.e., capable of undergoing (reversible) elongations in the direction of electricity conduction. Conductors of this type, which can be elongated along the main direction of electrical conduction while maintaining the conductive properties thereof, are defined in the present text and in the claims as stretchable conductive devices or even simply stretchable conductors.

Although the conductors of this type can be used in any situation in which a conductor is required, the intended main application thereof is in the production of electrodes implantable in the human (and animal) body, which requires that said electrodes can follow all the deformations of the part in which they are inserted, thus including elongations and returns to the initial length, without losing continuity and the main electrical features. This category includes, for example, implantable neural interfaces (described for example in WO 2009/090398 A2), deep brain stimulation devices (described for example in WO 2008/035344 A2), electrical stimulation devices of the spine for treating paralysis, and actuators in general, which are capable, for example, of stimulating or replacing muscle movement (known as "artificial muscles"). Given the importance of the latter application, in the remainder of the description reference will be made to implantable products and devices, but it is understood that the products of the invention are also applicable in all other situations in which a stretchable conductor is required.

A method for the preparation of an implantable device is also disclosed in US7085605.

A first proposed methodology for producing conductors with these features consists in preparing metallic lines (wires or thin deposits) with a wavy pattern inside biocompatible elastomeric polymers, making one or more electrical contacts emerge at the surface of the polymer at predefined points depending on the intended application; when the polymer undergoes an elongation, the wavy shape of the metallic line allows the elongation or shortening thereof. Conductors of this type are described for example in patents US 7,085,605 B2 and US 7,265,298 B2. However, the methods of these patents are not entirely satisfactory. Firstly, they are quite laborious and therefore not suitable for the transfer to production on an industrial scale; secondly, the products obtained with these methods are resistant to traction only in the average direction of the track (i.e., in the median direction of the undulation or corrugation).

A second approach is described in patent US 9,107,592 B2, and consists in depositing (with known methods) metallic tracks on a pre-stressed elastomer; after deposition, the elastomer is allowed to return to its size "at rest" and the metallic deposit geometrically rearranges to follow the contraction thereof. In this case, however, the metallic deposit is compressed in the resting elastomer; this can first involve a variation in the mechanical properties of the surface of the elastomer on which the metallic deposit is formed, which can induce the fracturing thereof during the repeated elongation and relaxation cycles to which the product will be subjected. Furthermore, also the products obtained with these methods are resistant to traction only in the direction along which the elastomer was initially pre-tensioned, and for a maximum extension equal to such a pre-tensioning.

Another approach is described in international patent application WO 2011/121017 A1 to the present Applicant. According to this method, the conductive line is created by implanting nano-sized aggregates of metals (for example, titanium) in an elastic polymer; the examples reported in the application demonstrate that, despite the deposit consisting of discrete particles, electrical continuity is guaranteed, as well as the maintenance thereof even after tens of thousands of elongation/shortening cycles of the conductor. The process described in this application also comprises the possibility of growing a continuous metallic layer, for example by electrochemical (galvanic) deposition, on top of the deposit obtained by nanoparticle implantation if this emerges at the surface of the support; in this case, the conductive layer obtained by particle implantation allows the connection to an external electrical circuit to provide the electrons necessary for electrochemical deposition. However, the method of this document is not easily scalable for industrial production.

Finally, a last method for producing conductive deposits on elastomeric supports is by means of deposition techniques such as Physical Vapor Deposition (PVD), in particular the variant known as Electron-beam evaporation which allows high deposition rates. This technique, and the results obtained therewith, are described for example in the articles "Stretchable conductors: thin gold films on silicone elastomer", S. P. Lacour et al., Mat. Res. Soc. Symp. Proc. Vol. 795 (2004); "Extended cyclic uniaxial loading of stretchable gold thin-films on elastomeric substrates", I. M. Graz et al., Applied Physics Letters 94, 071902 (2009); "Stretchable gold conductors embedded in PDMS and patterned by photolithography: fabrication and electromechanical characterization", T. Adrega et al., J. Micromech. Microeng. 20

(2010) 055025; and "Mechanisms of reversible stretchability of thin metal films on elastomeric substrates", S. P. Lacour et al., Applied Physics Letters 88, 204103 (2006). In all these articles, the system produced is always formed by a gold deposit on a silicone support, and it is not clear whether the described method is also applicable to other systems, in particular with metals other than gold; furthermore, to ensure the adhesion of the gold deposit to the elastomer, it is necessary to deposit an intermediate thin chromium layer, which makes these systems not ideal for implant applications in the human or animal body; finally, the metal films obtained with these techniques can only be elongated when they have very low thicknesses, as recognized in the aforementioned article "Stretchable conductors: thin gold films on silicone elastomer", in which the abstract reports that deposits with a thickness greater than 100 nm electrically break with a traction deformation of ~ 1%

### SUMMARY OF THE INVENTION

It is the object of the present invention to provide a method for the production of stretchable conductive devices which overcomes the problems of the background art, as well as to provide the devices obtained by means of said method.

This and other objects are achieved with the present invention, which in a first aspect relates to a method for the production of stretchable conductive devices comprising the following steps:
a) preparing an elastomeric support on a surface of which there is a first metallic deposit with a thickness between 10 and 200 nm obtained with a dry method;
b) forming a second metallic deposit on top of said first metallic deposit by chemical or electrochemical deposition from a solution;
characterized in that said deposition from a solution is carried out simultaneously to, or is followed by, an ultrasound treatment.

In a second aspect thereof, the invention relates to a stretchable conductive device formed by an elastomeric support on a surface of which there are one or more discontinuous metallic deposits but in which the parts from which they are made are in contact with each other.

### BRIEF DESCRIPTION OF THE FIGURES

Figure 1 depicts a photograph obtained with a scanning electron microscope (SEM) of a sample of the invention with a gold deposit at rest (Fig. 1.a), and three photographs at different magnifications of the same sample subjected to elongation (Fig. 1.b-1.d);
Figure 2 shows a top view of a device produced with the method of the invention, in which four metal tracks of gold are present on an elastomeric support;
Figure 3 shows the geometry of the metallic deposit of samples prepared for the measurement of resistance as a function of elongation/relaxation cycles;
Figure 4 depicts a photograph obtained with an optical microscope of the surface of a sample of the invention with a gold deposit;
Figure 5 depicts a photograph obtained with the same instrument and at the same magnification as Figure 4, on a sample not of the invention;
Figure 6 depicts a photograph obtained with an optical microscope of the surface of a sample of the invention with a platinum deposit;
Figure 7 depicts a photograph obtained with an optical microscope of the surface of a sample of the invention with an iridium deposition;
Figure 8 is a photograph of an apparatus for performing resistivity measurements during elongation/relaxation cycles;
Figure 9 shows a graph of the trend of surface resistivity values (Ω/□) as a function of % elongation for samples obtained with gold, platinum and iridium deposits.
Figure 10 shows a graph of the trend of surface resistivity values (Ω/□) as a function of elongation % for two gold deposit samples obtained according to the method of the invention, and of one gold deposit sample not obtained according to the method of the invention.

### DETAILED DESCRIPTION OF THE INVENTION

The Applicant has surprisingly found that by carrying out a chemical or electrochemical deposition from a solution of a metallic film on the surface of an elastomer, and treating the elastomeric support with ultrasound during or after said deposition, the deposit which is obtained is fractured, but the portions forming it are in contact with each other when the elastomeric support is at rest, and remain in contact at least for fractions of the edges thereof when the elastomeric support is deformed in elongation, thus maintaining its electrical conductivity features.

In the first aspect thereof, the invention relates to the method for producing stretchable conductive devices.

The first step of the method, a), consists in providing a support made of an elastomeric polymer on a surface of which a first metallic deposit obtained with a dry method is present.

The polymeric material of the support can be any elastomeric polymer, for example polyolefin-based elastomers, elastomeric fluoropolymers, polybutadiene (BR), styrene-butadiene rubbers (SBR), ethylene-propylene rubbers (EPR), ethylene-propylene-diene rubbers (EPDM), nitrile rubbers (NBR), acrylic rubbers (ACM), isobutylene-isoprene rubbers (IIR), co-polyesters, neoprene (polychloroprene), polyurethane rubbers and polysiloxanes (silicones). For the preferred object of the invention, i.e., making devices intended for implantation in the human or animal body, the polymer must be biocompatible; in this case the preferred material is a silicone, and polydimethylsiloxane, known in the field with the abbreviation PDMS, is particularly preferred.

The first metallic deposit can be produced with any technique known for the purpose, for example those described above in the discussion of the background art. These known techniques, with which the first deposit can be produced, are all of the "dry" type, and fall into the two general categories of evaporations or implantations. In particular, the first deposit can be produced with chemical deposition techniques such as Chemical Vapor Deposition (CVD), by means of physical deposition techniques such as thermal evaporation, Electron-beam evaporation or sputtering, or by means of cluster deposition techniques such as CBD (Cluster Beam Deposition), SCBD (Supersonic Cluster Beam Deposition) or SCBI (Supersonic Cluster Beam Implantation).

The CVD technique is widely known in the material science field and consists in thermally decomposing on the surface of a support (possibly masked to obtain a deposit having a desired geometry) a volatile compound, generally a metal-organic compound, of the metal of interest.

The physical techniques of evaporation or sputtering are widely known in the field of materials science and do not require a description herein.

The preferred technique according to the present invention to form the first metallic deposit is the technique known as Supersonic Cluster Beam Implantation (SCBI); in this case the first deposit is produced in the form of a layer of nanoparticles. The modes for the production of nanoparticle layers on the surface of elastomers with the latter technique are extensively described in the cited application WO 2011/121017 A1, to which reference is made for details. **In** short, the technique comprises the steps of: creating a beam of nano-sized neutral aggregates of a desired material, in which said aggregates have average speed between 100 and 10,000 m/s and dimensions less than 50 nm; and impinging the beam on said surface of an elastomeric material in a vacuum chamber. The SCBI technique is preferred in the present invention because the inventors have noted that a first deposit in the form of nanoparticles is more effective, as compared to continuous deposits, in then promoting the formation of the second deposit, in particular when this is produced by means of chemical deposition.

The advantage of the present invention as compared to the sole use of the dry techniques illustrated above is that these latter have relatively low deposition yields, in terms of thickness of deposit formed in the unit of time; therefore, obtaining thicknesses with these techniques such as to have resistivity values useful for practical purposes would require overly long times for industrial production. Vice versa, with the present invention, the above techniques to form the first deposit are used only for short times to produce metallic deposits of low thickness, on which the second deposit, of greater thickness, is then grown with techniques having higher yields.

Furthermore, the films obtained with some of the techniques of the background art, for example those of the mentioned papers to Lacour *et al.,* are not stretchable for thicknesses greater than about 100 nm; however, this involves high resistivity values and therefore limited applicability of the resulting films.

Furthermore, the greater thicknesses allowed by the invention give rise to greater resistance to corrosion phenomena over time.

The inventors have observed that the devices of the invention can be subjected to elongations of up to 70% of their length at rest, for thousands of cycles, without giving rise to performance decay.

The metals useful to form the first deposit can be different depending on the intended use of the final device. For general applications, essentially all transition metals can be employed as long as they are chemically resistant in the solution of the chemical or electrochemical deposition bath of the following operation of the method of the invention. In the case of articles intended for implantation in the human body, even if the first deposit is then covered by a second metallic deposit, it is preferable to use inert or biocompatible metals, in particular noble metals or titanium. The preferred metal for making this first deposit, both in continuous form and in the form of a layer of nanoparticles, is platinum.

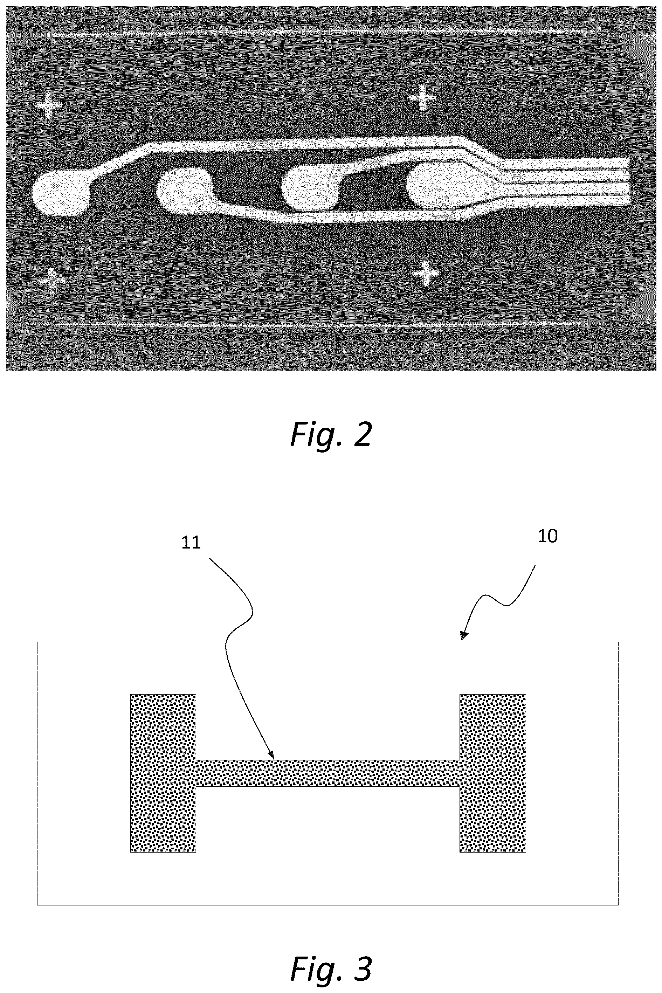

The formation of the first deposit can occur uniformly on the surface of the elastomeric support when the final device is intended for general use as a stretchable conductor. For most applications, and in particular for devices implantable in the body, however, it is preferable that the first deposit is in the form of tracks which allow separately and selectively conveying different electrical signals to different points of the device. The formation of metallic deposits with geometry having the form of tracks can be obtained with well-known methods derived from the semiconductor industry, i.e., by means of the use of stencil masks or with lithographic techniques, by means of the deposition and selective removal of layers of polymeric materials which can then be eliminated at the end of the deposition process of the desired material. In the case of devices intended for implantation in the body, the very high resolutions of semiconductor devices (lateral dimensions of the tracks or structures of the order of one micron or less) are normally not necessary, and the tracks can have lateral dimensions in the order of millimeters or tenths of a millimeter, which can also be obtained with stencil masks. Figure 2 shows an example of a device produced with the method of the invention, in which four distinct metal tracks are present on an elastomeric support: the minimum width of the tracks is 0.7 mm, and in the points of greatest proximity these are separated by spaces of width 0.3 mm.

Since the first metallic deposit does not have the function of primary electricity conductor in the final device, but only those of deposition electrode in the subsequent electrochemical deposition and of anchoring the metallic deposit to the elastomeric support (whether the metallic deposit is obtained by chemical or electrochemical deposition), this first deposit has a sub-micrometric thickness, and preferably between 10 and 200 nm.

Step a) can be followed, especially if much time elapses before the next step b) is carried out, by a chemical cleaning operation of the first deposit (analogous to the pickling operations of the metal industry), which can be carried out with reducing agents such as formic acid, hydrazine, alcohols or the like. This operation has the purpose of removing passivation layers from the metal surface of the first deposit due to exposure to air, and in the case of first deposits obtained by means of SCBI, it is also useful in the case of nanoparticles of noble metals, which, due to the enormous surface area, have a higher reactivity compared to the "bulk" versions of the same metals.

The second step of the method, b), consists in forming on the first metallic deposit obtained in the previous step a second metallic deposit by chemical or electrochemical deposition from a solution of a precursor of the desired metal; the second metallic deposit can be formed simultaneously with the application of ultrasound, or it can be formed under static conditions and then carrying out an ultrasound treatment of the deposit thus formed.

In this step it is possible to deposit a single metal, or a mixture of two metals if the chemical or electrochemical features thereof are similar.

Chemical deposition from a solution is known in the field as "Electroless Deposition" or its abbreviation "ELD", which will be adopted below.

This mode is carried out by immersing the elastomeric substrate on which the surface layer of the first deposit is present in a bath containing a salt or a complex of the metal with which the second deposit is to be formed; in the same bath (solution) a reducing agent is added, capable of providing electrons for a general reaction of the type

Mⁿ⁺ + n e- → M⁰

in which the metal, initially in the form of an ion in the n+ oxidation state (free, solvated or in the form of a complex), is reduced to a neutral metal.

In this case, the first metallic deposit on the support acts as a catalyst or nucleation center of the metal reduction reaction; therefore, the reduction with formation of the second metallic deposit occurs only at the first deposit.

The following are examples of reduction reactions of metal ions or complexes to the corresponding metal, respectively for gold, platinum and iridium:

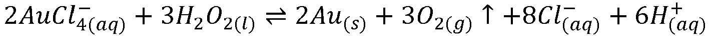

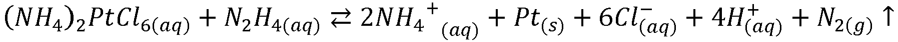

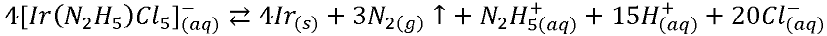

In these reactions, the reducing agent in the case of gold is hydrogen peroxide, while in the case of platinum and iridium it is hydrazine. In the above reactions for platinum and iridium, the complexes indicated as starting reagents are produced in situ by reaction of hexachloroplatinic acid with ammonia in the presence of hydrazine in the case of platinum, and by reaction of hexachloroiridic acid with hydrazine in the case of iridium.

ELD deposition can occur at temperatures between 5 and 80 °C, preferably between 15 and 70 °C, with a concentration of the metal ion to be reduced (free, solvated or in complex form) between 0.01 and 10 g/L. The concentration of the reducing agent varies as a function of the concentration of the metal ion, and the reductant is generally used in stoichiometric excess, typically in a molar ratio between 1:10 and 1:1000 with respect to the metal ion to be reduced. The deposition reaction typically has a duration between about 10 minutes and one hour.

Various additives can also be present in the solution.

Some additives which can be used are for example:
- surfactants, which act as brighteners of the metallic deposit, improve the diffusion of ultrasound in the deposition solution, and inhibit chemical deposition in solution; it is believed that these compounds act by "incorporating" through micellar or similar structures the metal nuclei which may form in solution, isolating them from the solution and preventing them from growing further and causing the electrochemical bath to collapse. Preferred surfactants for the purposes of the present invention are polyvinyl alcohols, in particular the products of the Mowiol^{®} series (trademark of Kuraray Specialties Europe GmbH);
- halogen ions, generally added through a salt of the metal to be deposited, which act as regulators of the reduction rate;
- or pH regulators, such as NaOH or HCl, in turn regulators of the reduction rate.

Electrochemical deposition from a solution is instead the technique well known as galvanic deposition, in which the electrons necessary for the reduction of the metal ion of interest are provided by an external electrical circuit; this deposition technique is also indicated in the field with the abbreviation ED (from "Electrodeposition"). In this case, the first deposit present on the support is connected to the external circuit and acts as an electrode on which the reduction occurs; also in this case, of course, the second metallic deposit is formed in correspondence of the first deposit, whether continuous or in the form of tracks.

The electrochemical deposition of metals, and bath compositions useful for the deposition of various metals, are widely known in the art and do not require a detailed description herein. The same baths described above for chemical deposition can also be used for electrochemical deposition, without the addition of the chemical reducing agent.

Electrochemical deposition can be carried out in a two or three electrode system.

A three-electrode cell consists of a working electrode, which in the case of the invention is the first metallic deposit on the elastomeric support; a reference electrode, which allows controlling the potential of the cell with respect to an external potential reference; and a counter-electrode which acts as a current collector.

A two-electrode cell is similar to the previous one, but in this case the reference electrode and the counter-electrode are short-circuited. In this case it is possible to control the current circulating in the cell, but the potential that is read is not linked to an external reference.

In the case of both ELD and ED, the thickness of the second metallic deposit is preferably between 50 and 1000 nm; with lower thicknesses the resistivity of the metallic deposit is high due to the low section thereof, while with higher thicknesses the elastic deformability of the device worsens.

The characteristic element of the present invention is the application of ultrasound for the formation of the second metallic deposit: both in the case of electroless deposition and in the case of electrochemical deposition, ultrasound can be applied to the solution during the deposition operation, or afterwards, by means of a treatment of the second deposit obtained by ELD or ED. In order to obtain an effective ultrasound action, the sample must be immersed in a liquid: in case ultrasound is applied during ELD or ED deposition, the liquid is naturally the deposition bath itself; in case ultrasound is instead applied after the formation of the second metallic deposit, the liquid can be any liquid phase, as long as it is chemically compatible with the polymer of the support and with the metals of the first and second deposits; in the case of devices intended for implantation in the body, the finished device is preferably rinsed with distilled water to eliminate traces of process solvents or chemical species dissolved therein.

Whether ultrasound is applied during or after the formation of the second metallic deposit, the effect is to obtain a micro-fractured deposit; this is the feature which allows maintaining the electrical conductivity of the obtained devices even after elongation of the sample.

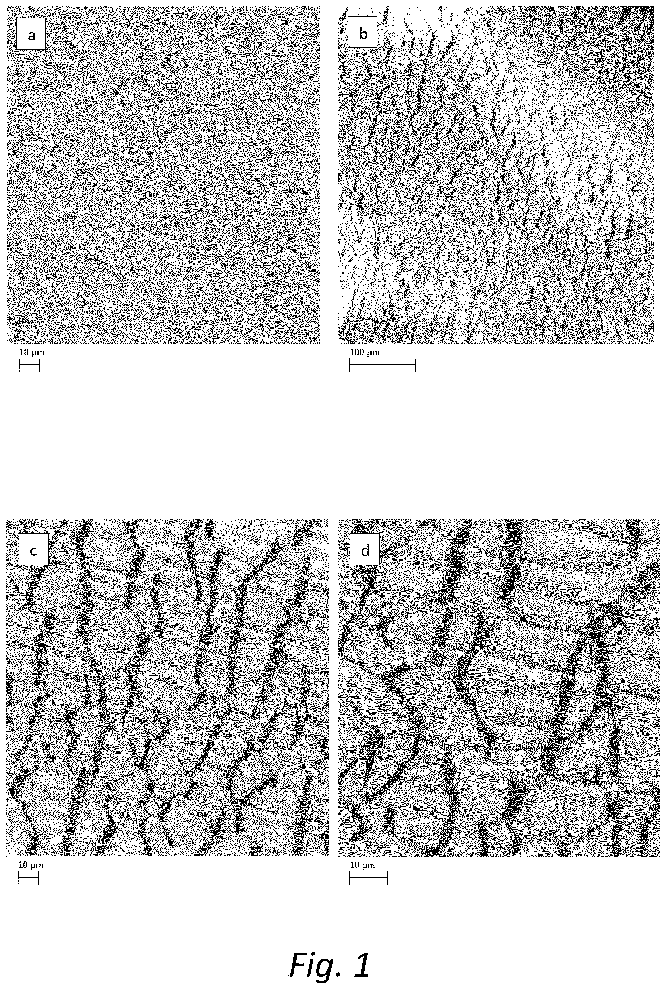

The phenomenon is shown in Figure 1. The images in Figures 1.a-1.d are reproductions of SEM photographs of a sample of a gold deposit on silicone, obtained with the use of ultrasound during deposition carried out by ELD. Figure 1.a shows the sample at rest, i.e., not subjected to stretching, while Figures 1.b-1.d show, at increasing magnifications, the same sample subjected to stretching in the direction from left to right in the photographs. As can be seen in Figure 1.a, the metallic deposit uniformly covers the sample surface, but the film appears fractured and in the form of "islands" in contact along all the edges thereof; upon stretching (Figures 1.b-1.d), the deposit "islands" move away, exposing the underlying elastomeric support (darker areas in the photographs), but there are always points of contact between said islands which form a continuous percolation path parallel to the support surface, maintaining the electrical conductivity of the deposit even when subjected to stretching. Figure 1.d shows, by means of the added dashed lines, some possible continuous paths which allow the passage of the electric charge, from right to left in the figure, in the elongation direction of the sample.

The inventors have observed that the range of ultrasound frequencies useful for the purposes of the invention is between 25 and 80 kHz: at frequencies below 25 kHz a strong cavitation effect occurs in the liquid in which the second deposit is immersed, in formation or already formed, and there is a high risk of damaging the sample; at frequencies above 80 kHz the cavitation is weak and the sample could not have the microcracks necessary to obtain the result of maintaining conductivity under stretching. Optimal ultrasound frequency values are between 30 and 50 kHz, preferably about 40 kHz.

Preferably, during the application of ultrasound the support is moved with respect to an average position, to obtain a uniform treatment of the second metallic deposit in formation or already formed; in fact, in some cases standing wave conditions could be achieved in the liquid, in which the position of the wave nodes remains constant over time, thus having areas of the sample, corresponding to these nodes, in which there is practically no treatment with the ultrasound. The movement of the support can be linear and reciprocating, with speeds between 5 mm/s and 20 mm/s. Alternatively, the support can be rotated with a speed between 10 and 50 rpm, preferably between 20 and 30 rpm, around an axis thereof. To further improve the uniformity of the ultrasound field on the surface of the support, the linear and rotational movements can be combined.

The invention will be further illustrated by the following Examples.

### MATERIALS, INSTRUMENTS, AND METHODS

The following materials were used for performing the tests of the examples:
- NuSil^{®} MED silicones, 6033, 4930, 4950, 4970 and 6640 series, produced and sold by the company Avantor^{™};
- LSR 4370 silicone of the company Elkem Siliconi Italia s.r.l. of Caronno Pertusella (VA);
- the tetrachloroauric, hexachloroplatinic and hexachloroiridic acids were purchased from METALOR Technologies SA of Marin (Switzerland);
- Mowiol^{®} 4-88 was purchased from Sigma-Aldrich;
- the first metallic deposits formed in the silicone supports, on which the second deposits were then grown, were in the form of layers of nanoparticles produced by means of SCBI technique, according to the methods described in patent application WO 2011/121017 A1 of the Applicant; in all cases the metal of the nanoparticles was Pt;
- the cell used for the production of the metallic deposits was a self-constructed three-electrode cell, with a reference electrode Ag/AgCl IS-AG/AGCL.AQ.RE.1 (produced and sold by PalmSens BV, Netherlands) and a graphite counter-electrode BASI-MW-4131 (PalmSens BV); the electrodes were connected to a PalmSens-04 potentiostat;
- the resistivity measurements were carried out with an Agilent 34410A/11A 6 ½ Digit Multimeter;
- an Elmasonic P 120 H bath sold by Avantor^{™} was used for generating ultrasound;
- the SEM images were obtained with a Zeiss LEO 1525 instrument;
- optical microscope images were obtained with an ASH OMNI 3 instrument.

### EXAMPLE 1

This example relates to the production of a gold deposit by the electroless method with ultrasound applied during deposition.

A silicone support was produced using NuSil^{®} MED 6033 precursor polymerized at 115 °C for 1 hour. The support had a thickness of 0.1 mm and lateral dimensions of 20 x 40 mm.

A layer of Pt nanoparticles was formed on one face of this support. The deposit had the geometry shown in Figure 3, obtained with a stencil mask. The deposit 11 obtained on the support 10 had the shape shown in the figure, known in the art as a "dog bone" shape, consisting of two lateral parts of greater area (5 x 12 mm each) connected by a central part 20 mm long and 1.5 mm wide. This shape is suitable for the stress resistance measurements which will be carried out later on the samples, with the two large lateral parts which allow the sample to be easily electrically connected to the clamps of a resistance meter, and the central part on which the stretching stress is focused.

The obtained nanoparticle layer had an average thickness of 63 nm, determined measuring by means of a stylus profilometer (KLA Tencor P-17, resolution up to 0.5 Å) the thickness of corresponding layers deposited on silicon substrates.

A gold deposition bath was prepared by adding into a beaker 35 mL of a 0.2 g/L concentration solution of tetrachloroauric acid, to which hydrogen peroxide was added until a 0.002 M concentration of this compound was obtained. The temperature was maintained at 35 °C by means of a thermostatic bath.

The previously prepared support was suspended with a wire and moved in the bath vertically with a 10 mm excursion at a speed of 10 mm/s, while ultrasound was applied to the bath at a frequency of 37 kHz.

The procedure was continued for 15 minutes.

The gold deposition occurred only in the areas where the layer of Pt nanoparticles had previously been formed, and therefore the final deposit had the same geometry shown in Figure 3.

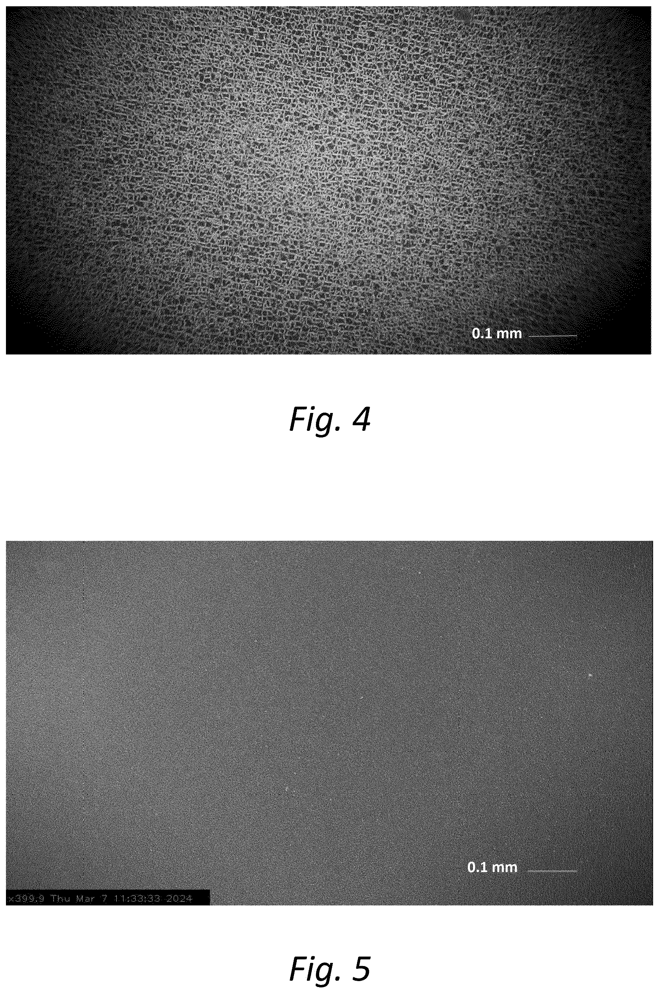

The sample obtained was examined under an optical microscope, obtaining the micrograph depicted in Figure 4.

In the figure, the lighter lines represent the fracture lines between the various islands of the gold deposit, while the dark areas are the islands of the deposit, i.e., internally continuous parts therein.

### EXAMPLE 2 (COMPARATIVE)

The test of Example 1 was repeated identically with the only difference that during the formation of the gold deposit, no ultrasound was applied to the deposition bath.

Figure 5 shows a micrograph obtained at the same magnifications as Figure 4: as can be noted, in this case the surface of the deposit has an essentially uniform and non-micro-cracked appearance like the deposit obtained in Example 1.

### EXAMPLE 3

This example relates to the production of a platinum deposit by electroless deposition with ultrasound applied during deposition.

The procedure of Example 1 was repeated until the Pt nanoparticle layer was obtained on the silicone support.

The support thus prepared was introduced into a bath maintained at 35 °C comprising hexachloroplatinic acid at a concentration of 0.3 g/L, hydrazine chloride at a concentration of 2.5 g/L, ammonia at a concentration of 35 g/L and potassium chloride at a concentration of 15 g/L.

During the platinum deposition, ultrasound was applied in the bath at 37 kHz and the support was moved vertically with a 10 mm excursion at a speed of 10 mm/s, and simultaneously rotated at 25 rpm. The deposition lasted 25 minutes.

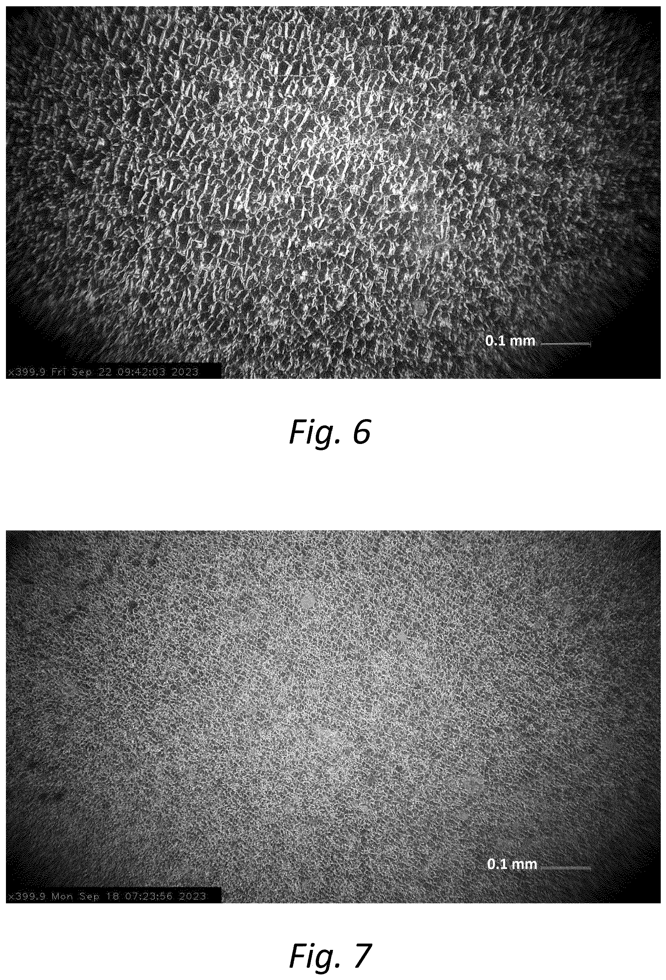

Figure 6 depicts a photograph obtained under an optical microscope showing the surface of the metallic deposit: as noted, the surface has the same micro-fractured structure as the gold sample in Figure 4.

### EXAMPLE 4

This example relates to the production of an iridium deposit by the electroless method with ultrasound applied during deposition.

The procedure of Example 3 was repeated, using however a bath containing hexachloroiridic acid at a concentration of 0.2 g/L, hydrazine chloride at a concentration of 0.1 g/L and sodium hydroxide at a concentration of 0.005 g/L; during the deposition of the iridium, which lasted 30 minutes, the bath was maintained at 70 °C.

Figure 7 depicts a photograph obtained under an optical microscope showing the surface of the metallic deposit: as noted, the surface has the same micro-fractured structure as the gold sample in Figure 4.

### EXAMPLE 5

This example relates to the production of a gold deposit by the electroless method with ultrasound applied after deposition.

The procedure of Example 1 was repeated until the Pt nanoparticle layer was obtained on the silicone support.

The support thus prepared was introduced into a bath maintained at 25 °C comprising tetrachloroauric acid at a concentration of 0.09 g/L, hydrogen peroxide at a concentration of 0.44 M, potassium chloride at a concentration of 0.5 g/L and Mowiol^{®} 4-88 at a concentration of 2.0 g/L.

The deposition reaction lasted 30 minutes.

The sample was not moved during deposition, while the bath solution was stirred with a magnetic stirrer.

At the end of the gold deposition step, the sample was inserted into a second beaker containing deionized water and was moved vertically with a 10 mm excursion at a speed of 10 mm/s for 5 minutes applying ultrasound at a frequency of 37 kHz to the liquid phase.

The sample obtained had features similar to that obtained in Example 1.

### EXAMPLE 6

This example relates to the measurement of the electrical conductivity of samples of deposits of the invention in elongation/relaxation cycles.

The surface resistivity of the samples obtained in Examples 1, 3 and 4 was measured under elongation conditions and in the subsequent return to the starting size (relaxation).

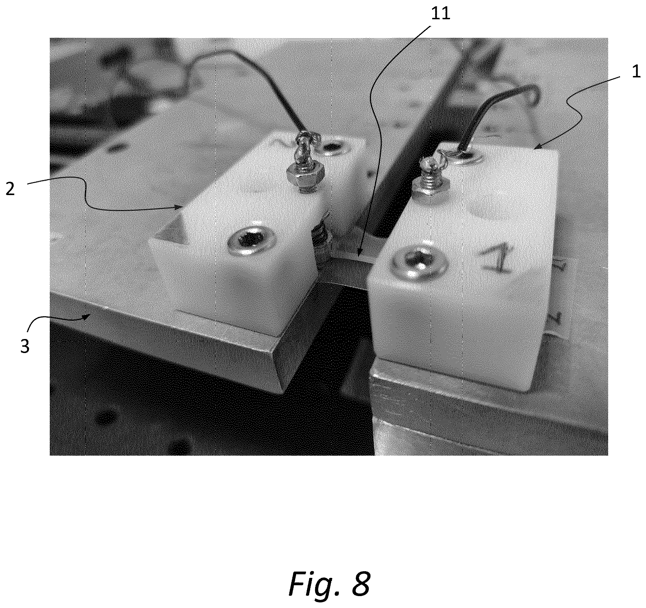

For the measurement of resistance as a function of traction, a self-constructed apparatus was used, shown in Figure 8, consisting of two electrically insulating clamps 1 and 2, in which clamp 1 was fixed on a sliding surface 3, so that it could be moved away from or closer to clamp 2. In the lower part of clamps 1 and 2 there are metal parts (not shown in the figure) which were used both to fix the sample for stretching, and to electrically connect it, through the lateral areas of the metallic deposit, to the resistance meter (multimeter); the central part of the deposit, which undergoes elongation during the test, is indicated in the figure with the reference number 11. A linear motor coupled to a resistance meter was used for the measurement. The movement of the movable surface 3 was controlled by a software edited with Labview; the tests consisted in performing 100 continuous cycles of elongation at a desired percentage of elongation and subsequent relaxation, simultaneously recording the resistance of the samples.

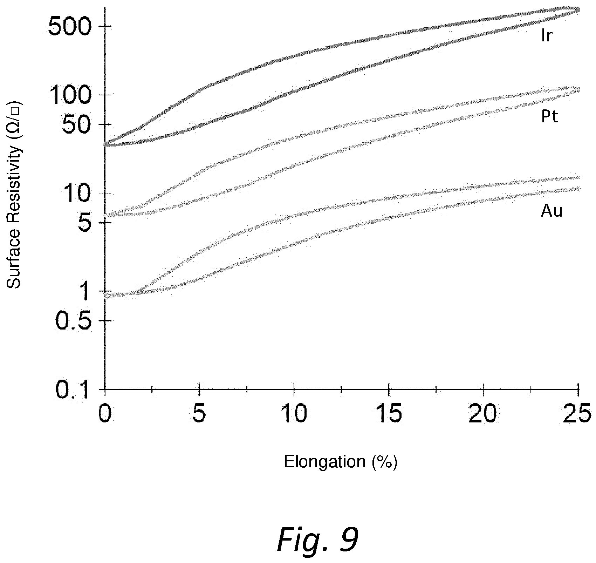

The results of the test are shown in Figure 9, which shows the surface resistivity values (Ω/□) as a function of the percentage elongation of the sample at the hundredth cycle.

### EXAMPLE 7 (COMPARATIVE)

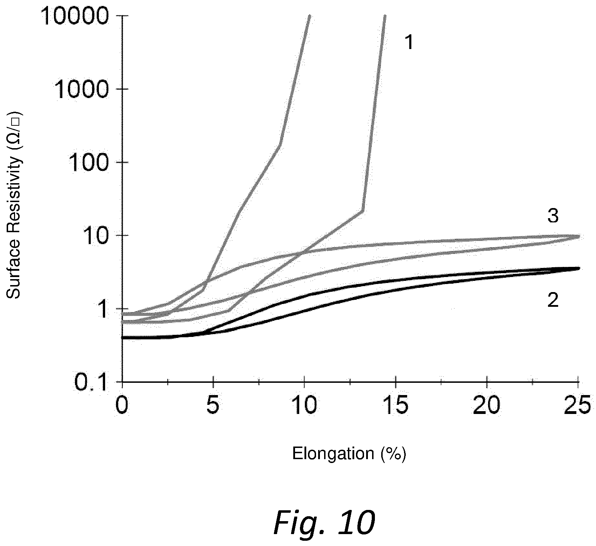

The test of Example 6 was repeated on the sample obtained in Example 2. The metallic deposit underwent electrical rupture at an elongation of less than 10%, as shown by curve 1 in Figure 10, which shows the surface resistivity values (Ω/□) of the sample as a function of percentage elongation; for comparison, the same figure shows the curves of the trend of the surface resistance of the sample of Example 1 (deposit of gold produced with ultrasound application during deposition, curve 2), and of a sample of Example 5 (gold deposit produced with ultrasonic application after deposition, curve 3).

## Claims

1. Method for the production of stretchable conductive devices which comprises the following steps:
a) preparing an elastomeric support on a surface of which there is a first metallic deposit with a thickness of between 10 and 200 nm obtained with a dry method;
b) forming a second metallic deposit on top of said first metallic deposit by chemical or electrochemical deposition from a solution;
**characterized in that** said deposition from a solution is carried out simultaneously to, or is followed by, an ultrasound treatment.

2. Method according to claim 1, wherein said elastomeric support is made with a polymeric material selected among polyolefin-based elastomers, elastomeric fluoropolymers, polybutadiene (BR), styrene-butadiene rubbers (SBR), ethylene-propylene rubbers (EPR), ethylene-propylene-diene rubbers (EPDM), nitrile rubbers (NBR), acrylic rubbers (ACM), isobutylene-isoprene rubbers (IIR), co-polyesters, neoprene (polychloroprene), polyurethane rubbers and polysiloxanes (silicones).

3. Method according to any one of claims 1 or 2, wherein said polymeric material is polydimethylsiloxane (PDMS).

4. Method according to any one of the preceding claims, wherein step a) is carried out with a dry method selected between an evaporation technique and a cluster deposition technique.

5. Method according to claim 4, wherein said evaporation technique is selected among Chemical Vapor Deposition (CVD), thermal evaporation, electron-beam evaporation and sputtering, and said cluster deposition technique is selected among CBD (Cluster Beam Deposition), SCBD (Supersonic Cluster Beam Deposition) and SCBI (Supersonic Cluster Beam Implantation).

6. Method according to any one of the preceding claims, wherein the metal of said first deposit is platinum.

7. Method according to any one of the preceding claims, wherein step b) is carried out by chemical reduction in solution of a salt or a complex of the metal of the second deposit by means of a reducing agent.

8. Method according to claim 7 in which the metal of the second deposit is chosen from gold, platinum and iridium; tetrachloroauric acid, hexachloroplatinic acid and hexachloroiridic acid are used respectively as precursors of the metals; and the reducing agent is hydrogen peroxide in the case of gold and hydrazine in the case of platinum and iridium.

9. Method according to any one of claims 7 and 8, wherein step b) is carried out at a temperature between 5 and 80 °C, preferably between 15 and 70 °C, with a concentration of the metal ion to be reduced between 0.01 and 10 g/L, and the reducing agent is used in a molar ratio between 1:10 and 1:1000 with respect to the metal ion to be reduced.

10. Method according to any one of claims 7 to 9, wherein the solution of step b) further comprises one or more additives selected from surfactants, halogen ions and **pH** regulators.

11. Method according to any one of claims 1 to 6, wherein step b) is carried out by electrochemical reduction in solution of a salt or a complex of the metal of the second deposit.

12. Method according to claim 11 in which the metal of the second deposit is selected among gold, platinum and iridium; and tetrachloroauric acid, hexachloroplatinic acid and hexachloroiridic acid are used respectively as precursors of the metals.

13. Method according to any one of claims 7 to 12, wherein the thickness of the second metallic deposit is of between 50 and 1000 nm.

14. Method according to any one of claims 7 to 13, wherein the frequency of the ultrasound applied during or after the formation of the second metallic deposit is between 25 and 80 kHz.

15. Method according to claim 14, wherein said frequency is between 30 and 50 kHz.

16. Method according to claim 15, wherein said frequency is about 40 kHz.

17. Method according to any one of claims 7 to 16, wherein during the application of ultrasound the support is moved linearly with respect to an average position with a speed ranging from 5 mm/s to 20 mm/s, and/or rotated with a speed between 10 and 50 rpm.

18. Method according to claim 17, wherein the speed at which the support is rotated is between 20 and 30 rpm.

19. Method according to any one of the preceding claims, in which between step a) and step b) a chemical cleaning operation of the first deposit is carried out using a reducing agent selected among formic acid, hydrazine and an alcohol.

## Patentansprüche

1. Verfahren zur Herstellung dehnbarer leitfähiger Vorrichtungen, das die folgenden Schritte umfasst:
a) Herstellen eines elastomeren Trägers, auf dessen Oberfläche sich eine erste metallische Ablagerung mit einer Dicke zwischen 10 und 200 nm befindet, die mit einem Trockenverfahren erhalten wurde;
b) Bilden einer zweiten metallischen Ablagerung auf der ersten metallischen Ablagerung durch chemische oder elektrochemische Abscheidung aus einer Lösung;
**dadurch gekennzeichnet, dass** die Abscheidung aus einer Lösung gleichzeitig mit einer Ultraschallbehandlung durchgeführt wird oder von einer solchen gefolgt wird.

2. Verfahren nach Anspruch 1, wobei der elastomere Träger aus einem Polymermaterial hergestellt ist, das ausgewählt ist aus Elastomeren auf Polyolefinbasis, elastomeren Fluorpolymeren, Polybutadien (BR), StyrolButadien-Kautschuken (SBR), Ethylen-Propylen-Kautschuken (EPR), Ethylen-Propylen-Dien-Kautschuken (EPDM), Nitrilkautschuken (NBR), Acrylkautschuken (ACM), Isobutylen-Isopren-Kautschuken (IIR), Copolyestern, Neopren (Polychloropren), Polyurethan-Kautschuken und Polysiloxanen (Silikonen).

3. Verfahren nach einem der Ansprüche 1 oder 2, wobei das Polymermaterial Polydimethylsiloxan (PDMS) ist.

4. Verfahren nach einem der vorhergehenden Ansprüche, wobei Schritt a) mit einem Trockenverfahren durchgeführt wird, das ausgewählt ist zwischen einer Verdampfungstechnik und einer Cluster-Abscheidungstechnik.

5. Verfahren nach Anspruch 4, wobei die Verdampfungstechnik ausgewählt ist aus chemischer Gasphasenabscheidung (CVD), thermischer Verdampfung, Elektronenstrahlverdampfung und Sputtern, und die Cluster-Abscheidungstechnik ausgewählt ist aus CBD (Cluster Beam Deposition, Clusterstrahlabscheidung), SCBD (Supersonic Cluster Beam Deposition, Überschall-Clusterstrahlabscheidung) und SCBI (Supersonic Cluster Beam Implantation, Überschall-Clusterstrahlimplantation).

6. Verfahren nach einem der vorhergehenden Ansprüche, wobei das Metall der ersten Ablagerung Platin ist.

7. Verfahren nach einem der vorhergehenden Ansprüche, wobei Schritt b) durch chemische Reduktion eines Salzes oder eines Komplexes des Metalls der zweiten Ablagerung in Lösung mittels eines Reduktionsmittels durchgeführt wird.

8. Verfahren nach Anspruch 7, bei dem das Metall der zweiten Ablagerung aus Gold, Platin und Iridium ausgewählt ist; Tetrachlorgoldsäure, Hexachloroplatinsäure bzw. Hexachloroiridiumsäure als Vorläufer der Metalle verwendet werden; und das Reduktionsmittel im Fall von Gold Wasserstoffperoxid und im Fall von Platin und Iridium Hydrazin ist.

9. Verfahren nach einem der Ansprüche 7 und 8, wobei Schritt b) bei einer Temperatur zwischen 5 und 80 °C, vorzugsweise zwischen 15 und 70 °C, mit einer Konzentration des zu reduzierenden Metallions zwischen 0,01 und 10 g/l durchgeführt wird und das Reduktionsmittel in einem Molverhältnis zwischen 1 : 10 und 1 : 1000 in Bezug auf das zu reduzierende Metallion verwendet wird.

10. Verfahren nach einem der Ansprüche 7 bis 9, wobei die Lösung aus Schritt b) ferner ein oder mehrere Additive umfasst, die aus Tensiden, Halogenionen und pH-Regulatoren ausgewählt sind.

11. Verfahren nach einem der Ansprüche 1 bis 6, wobei Schritt b) durch elektrochemische Reduktion eines Salzes oder eines Komplexes des Metalls der zweiten Ablagerung in Lösung durchgeführt wird.

12. Verfahren nach Anspruch 11, bei dem das Metall der zweiten Ablagerung aus Gold, Platin und Iridium ausgewählt ist; und Tetrachlorgoldsäure, Hexachloroplatinsäure bzw. Hexachloroiridiumsäure als Vorläufer der Metalle verwendet werden.

13. Verfahren nach einem der Ansprüche 7 bis 12, wobei die Dicke der zweiten metallischen Ablagerung zwischen 50 und 1000 nm liegt.

14. Verfahren nach einem der Ansprüche 7 bis 13, wobei die Frequenz des während oder nach der Bildung der zweiten metallischen Ablagerung angewendeten Ultraschalls zwischen 25 und 80 kHz liegt.

15. Verfahren nach Anspruch 14, wobei die Frequenz zwischen 30 und 50 kHz liegt.

16. Verfahren nach Anspruch 15, wobei die Frequenz etwa 40 kHz beträgt.

17. Verfahren nach einem der Ansprüche 7 bis 16, wobei der Träger während der Anwendung von Ultraschall linear in Bezug auf eine Durchschnittsposition mit einer Geschwindigkeit im Bereich von 5 mm/s bis 20 mm/s bewegt und/oder mit einer Geschwindigkeit zwischen 10 und 50 U/min gedreht wird.

18. Verfahren nach Anspruch 17, wobei die Geschwindigkeit, mit der der Träger gedreht wird, zwischen 20 und 30 U/min liegt.

19. Verfahren nach einem der vorhergehenden Ansprüche, bei dem zwischen Schritt a) und Schritt b) ein chemischer Reinigungsvorgang der ersten Ablagerung unter Verwendung eines Reduktionsmittels durchgeführt wird, das aus Ameisensäure, Hydrazin und einem Alkohol ausgewählt ist.

## Revendications

1. Procédé de production de dispositifs conducteurs étirables qui comprend les étapes suivantes :
a) préparer un support élastomère sur une surface duquel se trouve un premier dépôt métallique d'une épaisseur entre 10 et 200 nm obtenu par voie sèche ;
b) former un deuxième dépôt métallique au-dessus dudit premier dépôt métallique par dépôt chimique ou électrochimique à partir d'une solution ;
**caractérisé en ce que** ledit dépôt à partir d'une solution est réalisé simultanément à, ou est suivi d'un traitement par ultrasons.

2. Procédé selon la revendication 1, dans lequel ledit support élastomère est réalisé avec un matériau polymère sélectionné parmi les élastomères à base de polyoléfine, les polymères fluorés élastomères, le polybutadiène (BR), les caoutchoucs styrène-butadiène (SBR), les caoutchoucs éthylène-propylène (EPR), les caoutchoucs éthylène-propylène-diène (EPDM), les caoutchoucs nitrile (NBR), les caoutchoucs acryliques (ACM), les caoutchoucs isobutylène-isoprène (IIR), les copolyesters, le néoprène (polychloroprène), les caoutchoucs polyuréthane et les polysiloxanes (silicones).

3. Procédé selon l'une quelconque des revendications 1 ou 2, dans lequel ledit matériau polymère est le polydiméthylsiloxane (PDMS).

4. Procédé selon l'une quelconque des revendications précédentes, dans lequel l'étape a) est réalisée avec un procédé à sec sélectionné entre une technique d'évaporation et une technique de dépôt d'agrégats.

5. Procédé selon la revendication 4, dans lequel ladite technique d'évaporation est sélectionnée parmi le dépôt chimique en phase vapeur (en Anglais « Chemical Vapor Deposition » pour CVD), l'évaporation thermique, l'évaporation par faisceau électronique et la pulvérisation, et ladite technique de dépôt d'agrégats est sélectionnée parmi le CBD (en Anglais « Cluster Beam Deposition » pour dépôt par jet d'agrégats), le SCBD (en Anglais « Supersonic Cluster Beam Deposition » pour dépôt par jet d'agrégats supersonique) et le SCBI (en Anglais « Supersonic Cluster Beam Implantation » pour implantation par jet d'agrégats supersonique).

6. Procédé selon l'une quelconque des revendications précédentes, dans lequel le métal dudit premier dépôt est le platine.

7. Procédé selon l'une quelconque des revendications précédentes, dans lequel l'étape b) est réalisée par réduction chimique en solution d'un sel ou d'un complexe du métal du deuxième dépôt au moyen d'un agent réducteur.

8. Procédé selon la revendication 7, dans lequel le métal du deuxième dépôt est choisi parmi l'or, le platine et l'iridium ; l'acide tétrachloroaurique, l'acide hexachloroplatinique et l'acide hexachloroiridique sont utilisés respectivement comme précurseurs des métaux ; et l'agent réducteur est le peroxyde d'hydrogène dans le cas de l'or et l'hydrazine dans le cas du platine et de l'iridium.

9. Procédé selon l'une quelconque des revendications 7 et 8, dans lequel l'étape b) est réalisée à une température entre 5 et 80 °C, de préférence entre 15 et 70 °C, avec une concentration de l'ion métallique à réduire entre 0,01 et 10 g/L, et l'agent réducteur est utilisé dans un rapport molaire entre 1 : 10 et 1 : 1000 par rapport à l'ion métallique à réduire.

10. Procédé selon l'une quelconque des revendications 7 à 9, dans lequel la solution de l'étape b) comprend en outre un ou plusieurs additifs sélectionnés parmi les tensioactifs, les ions halogènes et les régulateurs de pH.

11. Procédé selon l'une quelconque des revendications 1 à 6, dans lequel l'étape b) est réalisée par réduction électrochimique en solution d'un sel ou d'un complexe du métal du deuxième dépôt.

12. Procédé selon la revendication 11, dans lequel le métal du deuxième dépôt est sélectionné parmi l'or, le platine et l'iridium ; et l'acide tétrachloroaurique, l'acide hexachloroplatinique et l'acide hexachloroiridique sont utilisés respectivement comme précurseurs des métaux.

13. Procédé selon l'une quelconque des revendications 7 à 12, dans lequel l'épaisseur du deuxième dépôt métallique est entre 50 et 1000 nm.

14. Procédé selon l'une quelconque des revendications 7 à 13, dans lequel la fréquence des ultrasons appliqués pendant ou après la formation du deuxième dépôt métallique est entre 25 et 80 kHz.

15. Procédé selon la revendication 14, dans lequel ladite fréquence est entre 30 et 50 kHz.

16. Procédé selon la revendication 15, dans lequel ladite fréquence est d'environ 40 kHz.

17. Procédé selon l'une quelconque des revendications 7 à 16, dans lequel pendant l'application des ultrasons, le support est déplacé linéairement par rapport à une position moyenne avec une vitesse allant de 5 mm/s à 20 mm/s, et/ou tourné avec une vitesse entre 10 et 50 tr/min.

18. Procédé selon la revendication 17, dans lequel la vitesse à laquelle le support est mis en rotation est entre 20 et 30 tr/min.

19. Procédé selon l'une quelconque des revendications précédentes, dans lequel entre l'étape a) et l'étape b), une opération de nettoyage chimique du premier dépôt est réalisée à l'aide d'un agent réducteur sélectionné parmi l'acide formique, l'hydrazine et un alcool.
